# FASCICULE DE BREVET EUROPEEN

(11) **EP 0 505 232 B1**
(45) Date de publication et mention de la délivrance du brevet: **02.11.1995**
(21) Numéro de dépôt: 92400601.8
(22) Date de dépôt: 09.03.1992
(51) Int. Cl.: A61M 16/00

(54) **Installation de fourniture de surpression de gaz respiratoire et procédé de commande d'une telle installation**
Vorrichtung zum Erzeugen von Überdruck in Atemgas sowie Verfahren zum Steuern dieser Vorrichtung
Apparatus providing respiratory gas overpressure, and a control process therefor

(30) Priorité: 21.03.1991 FR 9103431
(43) Date de publication de la demande: 23.09.1992
(73) Titulaire: TAEMA, F-92182 Antony Cédex (FR)
(72) Inventeur: Champain, Roger, F-78350 Les Loges en Josas (FR); Kissi, Nourredine, F-59176 Masny (FR); Zalkin, Daniel, F-78120 Rambouillet (FR)
(74) Mandataire: Le Moenner, Gabriel

(56) Documents cités:
- WO-A-88/10108
- WO-A-89/10768
- WO-A-90/14121
- US-A- 4 905 687
- BEHEBEHANI et.al "A microprocessor-based sleep apnea ventilator", 9 Novembre 1989,IEEE, WASHINGTON

## Description

La présente invention concerne un procédé de commande d'une installation de fourniture de surpression de gaz respiratoire comportant une turbine à faible inertie et des moyens de commande de la turbine fournissant un signal de commande à la turbine en fonction d'une valeur de consigne de surpression déterminée, notamment pour l'assistance respiratoire de personnes souffrant de troubles respiratoires, en particulier du sommeil.

Une installation et un procédé de commande du type ci-dessus sont décrits dans le document FR - A - 2 663 547 publié le 27-12-91, au nom de la Demanderesse. Dans l'installation connue, l'utilisation d'une turbine à faible inertie et dont la régulation de débit peut être effectuée en temps réel en fonction de la pression délivrée mesurée directement dans le masque de l'utilisateur permet de garantir à l'utilisateur une pression d'inspiration nécessaire à son confort respiratoire.

WO-A-90/14121 décrit un système du type susmentionné pour régler la pression de gaz respiratoire.

La présente invention a pour objet de proposer un procédé permettant, avec des moyens simples et fiables et de faibles coûts de fabrication, d'augmenter encore le confort respiratoire de l'utilisateur par application d'une faible augmentation de la surpression durant la phase inspiratoire de l'utilisateur.

Pour ce faire, selon une caractéristique du procédé selon l'invention, ce dernier comporte l'étape de détecter des variations périodiques du régime de la turbine et de modifier en conséquence la valeur de consigne pour moduler la surpression fournie par la turbine.

De façon plus spécifique, la détection des variations de régime de la turbine est effectuée par traitement du signal de commande de la turbine et affectation séquentielle aux moyens de commande de la turbine de deux valeurs de consignes différentes, en l'occurrence une consigne de pression inspiratoire (I) et une consigne de pression expiratoire (E) prédéterminées par l'opérateur.

La présente invention a pour autre objet une installation de fourniture de surpression de gaz respiratoire, du type comprenant une turbine à faible inertie et des moyens de commande de la turbine fournissant à la turbine un signal de commande en fonction d'une valeur de consigne de pression déterminée pour l'obtention d'une surpression délivrée déterminée comprenant des moyens de réglage, sensibles à des variations périodiques du régime de la turbine et couplés aux moyens de commande pour modifier temporairement les conditions de consigne et moduler la surpression fournie par la turbine.

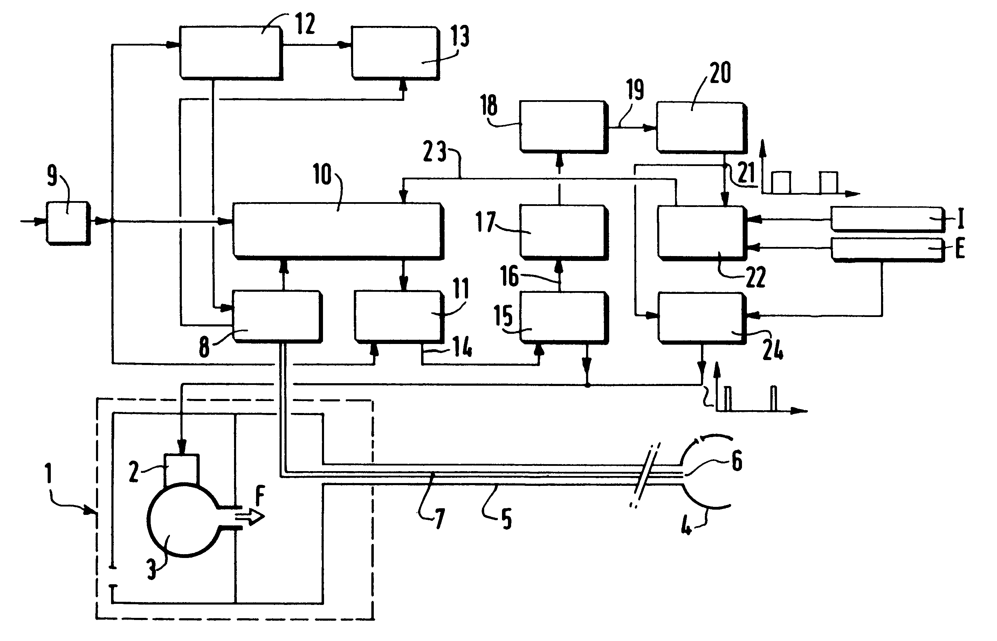

D'autres caractéristiques et avantages de la présente invention ressortiront de la description suivante d'un mode de réalisation, donné à titre illustratif mais nullement limitatif, faite en relation avec le dessin annexé, sur lequel :
- la figure unique représente schématiquement une installation de fourniture de surpression de gaz respiratoire selon l'invention.

On reconnaît, au bas de la figure unique, les éléments mécaniques de l'installation selon le document FR-A- 2 663 547 sus-mentionné , à savoir, dans un boîtier insonorisé 1, un bloc moteur 2/turbine 3 délivrant un flux de gaz respiratoire F en légère surpression, entre 4 et 20 HPa, à un masque utilisateur 4 via une conduite de gaz respiratoire 5, le masque 4 comportant une prise de pression 6 communiquant, via une tubulure 7, avec un capteur de pression piézoélectrique 8. L'installation comprend une carte électronique de commande et d'affichage,alimentée en courant continu 15 V, 2,5 A par un bloc d'alimentation 9, la carte comprenant une unité centrale de commande 10 et un module de puissance 11 relié au moteur 2 de la turbine 3, ainsi qu'un second bloc 12 d'alimentation à tension réduite alimentant le capteur de pression 8 et un module 13 à diodes électroluminescentes d'affichage de la pression délivrée à l'utilisateur.

Selon l'invention, la sortie 14 du module de puissance 11 est adressée à un module détecteur 15, en l'occurrence une faible résistance de l'ordre de 0,05 ohm, fournissant un signal 16 représentatif d'un début de phase inspiratoire ou d'un début de phase expiratoire de l'utilisateur puisque le régime de la turbine est adapté, par l'unité de commande 10, en fonction des variations de pression détectée dans le masque 4 par le capteur 8. Le signal 16 est filtré dans un filtre passe-bas 17 puis dérivé dans un étage de dérivation 18 qui fournit ainsi en sortie un signal 19 représentatif de la variation du débit de la turbine 3. Le signal 19 est adressé à un circuit de mise en forme 20 qui fournit un signal de sortie 21 à deux états 0 et 1.

L'installation selon l'invention comprend en outre un premier module réglable de consigne de pression inspiratoire I et un second module réglable de consigne de pression expiratoire E connectés à un module de commutation 22 affectant, par sa sortie 23, à l'unité de commande 10 l'une ou l'autre des valeurs de consigne I, E en fonction des états 0 et 1 du signal 21.

Comme sus-mentionné, cet agencement permet de modifier la valeur de consigne de la régulation de surpression imposée à l'utilisateur en fonction du cycle respiratoire de ce dernier. En effet, lorsque est détecté le début d'une phase inspiratoire, le seuil de régulation de la surpression fournie par la turbine est porté à une première valeur haute (I), augmentée de plusieurs centimètres d'eau, facilitant ainsi l'introduction d'air respiratoire dans les poumons de l'utilisateur. Par la suite, lorsqu'est détecté le début d'une phase expiratoire, le seuil de régulation de surpression est ramené à la valeur initiale (E), permettant ainsi une expiration plus facile grâce à un abaissement de la pression à laquelle les poumons de l'utilisateur sont exposés.

Avec l'agencement qui vient d'être décrit, la réponse de la turbine, grâce à sa faible inertie, est tout à fait satisfaisante pour la génération de l'augmentation de pression dès le début de la phase d'inspiration mais peut encore provoquer une légère gêne expiratoire lors de la commutation vers le seuil bas de pression (E), en fin d'inspiration. Conformément à un aspect de l'invention, pour améliorer le comportement de la turbine durant cette phase transitoire, il est prévu un module de freinage 24 recevant le signal 21 provenant du module de mise en forme 20 et le signal fourni par le second module de consigne de pression expiratoire E pour freiner temporairement la turbine et la ramener ainsi très rapidement à son régime correspondant à la fourniture de la surpression déterminée pour une phase expiratoire de l'utilisateur. De façon plus spécifique, le module 24 met temporairement le moteur 2 de la turbine 3 en court-circuit, la durée de mise en court-circuit étant fonction de la valeur de consigne basse du second module de consigne E et de la fréquence des cycles inspiration/expiration de l'utilisateur.

Quoique la présente invention ait été décrite en relation avec un mode de réalisation particulier, elle ne s'en trouve pas limitée mais est au contraire susceptible de modifications et de variantes qui apparaîtront à l'homme de l'art.

## Revendications

1. Procédé de commande d'une installation de fourniture de surpression de gaz respiratoire comportant une turbine à faible inertie (3) et des moyens (10) de commande de la turbine fournissant un signal de commande à la turbine en fonction d'une valeur de consigne de surpression déterminée comprenant l'étape de détecter (15) des variations périodiques du régime de la turbine, caractérisé en ce qu'il comprend l'étape de modifier en conséquence la valeur de consigne pour moduler la surpression fournie par la turbine.

2. Procédé selon la revendication 1, caractérisé en ce que la détection des variations de régime est effectuée par traitement (15, 17, 18, 20) du signal (14) de commande de la turbine (3).

3. Procédé selon la revendication 2, caractérisé en ce que le traitement du signal comprend les étapes suivantes : filtration (17), dérivation (18) et mise en forme (20) du signal détecté pour obtenir une suite de signaux d'états 0 et 1.

4. Procédé selon la revendication 3, caractérisé en ce qu'au passage à l'état 1, la valeur de consigne est commutée à une première valeur (I) pour fournir temporairement une surpression augmentée.

5. Procédé selon la revendication 4, caractérisé en ce qu'au passage à l'état 0, la valeur de consigne est ramenée à une seconde valeur inférieure (E).

6. Procédé selon la revendication 5, caractérisé en ce qu'il comprend en outre, au passage à l'état 0, une étape de freinage (24) de la turbine (3).

7. Procédé selon la revendication 6, caractérisé en ce que le freinage est obtenu par mise en court-circuit temporaire du moteur (2) d'entraînement de la turbine.

8. Installation de fourniture de surpression de gaz respiratoire, comprenant une turbine à faible inertie (3) et des moyens (10) de commande de la turbine, fournissant à la turbine un signal de commande (14) fonction d'une valeur de consigne de surpression déterminée (I, E) pour l'obtention d'une surpression délivrée déterminée comprenant des moyens (15, 17, 18, 20, 22) de réglage, sensibles à des variations périodiques du régime de la turbine, caractérisé en ce que les moyens de réglage sont couplés aux moyens de commande (10) pour modifier temporairement les conditions de consigne et moduler la surpression fournie par la turbine.

9. Installation selon la revendication 8, caractérisée en ce que les moyens de réglage comprennent des moyens (I, E) de génération de deux valeurs de consigne différentes et des moyens de traitement du signal de commande pour affecter (23) aux moyens de commande (10) l'une puis l'autre des deux valeurs de consigne.

10. Installation selon la revendication 9, caractérisée en ce que les moyens de traitement comprennent un étage de filtre (17), un étage de dérivation (18) et un étage (20) de mise en forme du signal détecté.

11. Installation selon l'une des revendications 8 à 10, caractérisée en ce qu'elle comprend en outre un moyen (24) de commande de freinage du moteur (2) de la turbine (3) agissant en réponse à l'affectation (22) aux moyens de commande (10) d'une (E) des valeurs de consigne.

## Claims

1. Method for controlling an installation for supplying pressurised respiratory gas, including a low-inertia turbine (3) and means (10) for controlling the turbine supplying a control signal to the turbine as a function of a given set pressure value, comprising the step of detecting (15) periodic variations in the speed of the turbine, characterised in that it comprises the step of modifying the set value accordingly in order to modulate the pressure supplied by the turbine.

2. Method according to Claim 1, characterised in that detection of the speed variations is effected by processing (15, 17, 18, 20) the signal (14) controlling the turbine (3).

3. Method according to Claim 2, characterised in that the processing of the signal comprises the following steps: filtering (17), differentiation (18) and shaping (20) of the signal detected in order to obtain a sequence of signals of states 0 and 1.

4. Method according to Claim 3, characterised in that, when going to state 1, the set value is switched to a first value (I) in order temporarily to supply an increased pressure.

5. Method according to Claim 4, characterised in that, when going to state 0, the set value is returned to a second, lower value (E).

6. Method according to Claim 5, characterised in that it also comprises, when going to state 0, a step of braking (24) the turbine (3).

7. Method according to Claim 6, characterised in that the braking is obtained by temporary short-circuiting of the motor (2) driving the turbine.

8. Installation for supplying pressurised respiratory gas, comprising a low-inertia turbine (3) and means (10) for controlling the turbine, supplying to the turbine a control signal (14) which is a function of a given set pressure value (I, E) for obtaining a given delivered pressure, comprising adjustment means (15, 17, 18, 20, 22), sensitive to periodic variations in the speed of the turbine, characterised in that the adjustment means are coupled to the control means (10) for temporarily modifying the set conditions and modulating the pressure supplied by the turbine.

9. Installation according to Claim 8, characterised in that the adjustment means comprise means (I, E) for generating two different set values and means of processing the control signal in order to allocate (23), to the control means (10), one and then the other of the two set values.

10. Installation according to Claim 9, characterised in that the processing means comprise a filter stage (17), a differentiation stage (18) and a stage (20) for shaping the signal detected.

11. Installation according to one of Claims 8 to 10, characterised in that it also comprises a means (24) for controlling the braking of the motor (2) of the turbine (3) acting in response to the allocation (22), to the control means (10), of one (E) of the set values.

## Patentansprüche

1. Verfahren zum Steuern einer Vorrichtung zum Erzeugen von Überdruck in Atemgas, welche eine Turbine mit geringer Trägheit (3) und Mittel (10) zum Steuern der Turbine, die der Turbine ein Steuersignal in Abhängigkeit von einem vorbestimmten Überdruck-Einstellwert zuführen, aufweist, umfassend den Schritt des Erfassens (15) periodischer Schwankungen der Turbinendrehzahl, gekennzeichnet durch den Schritt des dementsprechenden Änderns des Einstellwerts zum Ändern des durch die Turbine erzeugten Überdrucks.

2. Verfahren nach Anspruch 1, dadurch gekennzeichnet, daß die Erfassung der Drehzahlschwankungen durch Verarbeiten (15, 17, 18, 20) des Steuersignals (14) der Turbine (3) erfolgt.

3. Verfahren nach Anspruch 2, dadurch gekennzeichnet, daß das Verarbeiten des Signals die folgenden Schritte umfaßt: Filterung (17), Differenzierung (18) und Formung (20) des erfaßten Signals so, daß eine Signalfolge mit Zuständen 0 und 1 erhalten wird.

4. Verfahren nach Anspruch 3, dadurch gekennzeichnet, daß der Einstellwert beim Übergang auf den Zustand 1 auf einen ersten Wert (I) umgeschaltet wird, um vorübergehend einen erhöhten Überdruck zu erzeugen.

5. Verfahren nach Anspruch 4, dadurch gekennzeichnet, daß der Einstellwert beim Übergang auf den Zustand 0 wieder auf einen zweiten, kleineren Wert (E) verringert wird.

6. Verfahren nach Anspruch 5, weiter gekennzeichnet durch einen Schritt des Abbremsens (24) der Turbine (3) beim Übergang auf den Zustand 0.

7. Verfahren nach Anspruch 6, dadurch gekennzeichnet, daß das Abbremsen durch vorübergehendes Kurzschließen des Antriebsmotors (2) der Turbine erzielt wird.

8. Vorrichtung zum Erzeugen von Überdruck in Atemgas, umfassend eine Turbine mit geringer Trägheit (3), Mittel (10) zum Steuern der Turbine, die der Turbine ein Steuersignal (14) in Abhängigkeit von einem vorbestimmten Überdruck-Einstellwert (I, E) zuführen, um einen vorbestimmten abgegebenen Überdruck zu erhalten, und Einstellmittel (15, 17, 18, 20, 22), die auf periodische Drehzahlschwankungen der Turbine ansprechen, dadurch gekennzeichnet, daß die Einstellmittel mit den Steuermitteln (10) gekoppelt sind, um vorübergehend die Einstellzustände zu ändern und den durch die Turbine erzeugten Überdruck zu ändern.

9. Vorrichtung nach Anspruch 8, dadurch gekennzeichnet, daß die Einstellmittel Mittel (I, E) zum Erzeugen zweier verschiedener Einstellwerte und Mittel zur Verarbeitung des Steuersignals zum Beaufschlagen (23) der Steuermittel (10) mit dem einen und dann mit dem anderen der beiden Einstellwerte umfassen.

10. Vorrichtung nach Anspruch 9, dadurch gekennzeichnet, daß die Verarbeitungsmittel eine Filterstufe (17), eine Differenzierstufe (18) und eine Stufe (20) zur Formung des erfaßten Signals umfassen.

11. Vorrichtung nach einem der Ansprüche 8 bis 10, weiter gekennzeichnet durch ein Mittel (24) zur Steuerung des Abbremsens des Motors (2) der Turbine (3), welches in Antwort auf das Beaufschlagen (22) der Steuermittel (10) mit einem (E) der Einstellwerte wirkt.
